# EUROPEAN PATENT SPECIFICATION

(11) **EP 3 606 442 B1**
(45) Date of publication and mention of the grant of the patent: **13.05.2026**
(21) Application number: 18710888.1
(22) Date of filing: 19.03.2018
(51) Int. Cl.: A61B 17/00

(54) **DEVICE FOR CLOSING A TROCAR SITE**
VORRICHTUNG ZUM VERSCHLIESSEN EINER TROKARSTELLE
DISPOSITIF DE FERMETURE D'UN SITE DE TROCART

(30) Priority: 07.04.2017 IT 201700038786
(43) Date of publication of application: 12.02.2020
(73) Proprietor: Mascia Brunelli S.p.A., 20128 Milano (MI) (IT)
(72) Inventor: ANDRAOS, Youssef Assaad, Jal El Dib (LB)
(74) Representative: Ripamonti, Enrico
(86) International application number: PCT/EP2018/056829
(87) International publication number: WO 2018/184822

(56) References cited:
- WO-A1-2010/028300
- DE-A1- 102010 048 908
- US-A- 5 192 301
- US-A1- 2005 045 188
- US-A1- 2007 270 766

## Description

### FIELD OF THE INVENTION

The present invention refers to a device for closing a trocar site.

More specifically, it refers to a device for closing a trocar site after surgery of an intra-abdominal cavity, used in particular in laparoscopic surgeries.

### STATE OF THE ART

At present, about 90% of the intra-abdominal cavity surgical operations (for instance, digestive system, gynecologic, urologic, or vascular surgeries) takes place laparoscopically. In order to perform this type of surgery, a surgeon shall perform several incisions in the abdominal wall, so as to make it possible the introduction of the surgical instruments necessary for the type of surgical operation to be carried out.

The introduction of surgical instruments is performed with the aid of devices called trocars, which hold the desired incisions temporarily open. Such incisions are referred to as trocar sites.

At the end of the surgical operation, the surgeon shall remove the trocars from the abdominal wall of the patient and close the incisions, i.e. the trocar sites, to prevent complications such as bleeding, due to injuries to inner organs having taken place through a trocar sites, or herniations of inner organs. Such complications might lead to difficult, expensive, and lengthy corrective measures, such as blood transfusions, hernia repairs, or further surgical interventions.

At present, the trocar sites are closed by way of a direct and/or laparoscopic suture. These conventional suturing techniques are difficult to carry out, in particular with obese patients, irrespective of the type of surgical intervention, even more in case of bariatric surgery. Although the use of various laparoscopic instruments has made it easier to close the trocar sites, this type of operation remains in any way difficult for a surgeon, in that it entails non-negligible risks of complications, such as the onset of nevromas or chronic pains, or breakages of suture or muscle.

US 2007/270766 A1 relates to a portal plug that allows a surgeon to temporarily close a surgical portal while draining fluids from the soft tissue surrounding the surgical field.

DE 10 2010 048908 A1 relates to a compressible plug insertable to a treatment site via a trocar. A funnel shaped insertion aid is also provided.

### SUMMARY OF THE INVENTION

The invention is set out in the appended set of claims.

An object of the present invention is therefore to provide a device for closing trocar sites that allows to close such sites in a simple and safe manner.

A further object of the invention is to provide a device that allows to make the trocar site closing operations efficient, easy, fast, and reproducible for any surgeons.

A further object of the invention is to provide a device for closing trocar sites that is structurally simple, easy to use and to manufacture, and that can be used for any types of surgeries (for instance abdominal, thoracic, or arthroscopic surgeries).

These objects and others, which will be apparent to the persons skilled in the art, are achieved by a device for closing trocar sites implemented according to the technical teachings of the attached claims.

Advantageously, the device according to the invention comprises a so-called plug; more specifically, the device comprises a body made from a resorbable and sterile, biocompatible polymer, featuring an elongate shape along a longitudinal axis, and featuring a first and second extremal surfaces and at least one longitudinal surface, which extends along the longitudinal axis between the first and second extremal surfaces, at least a portion of the body taken longitudinally between these extremal surfaces being solid.

Further characteristics and advantages of the invention will be apparent from the description of a preferred but not exclusive embodiment of the device for closing a trocar site, illustrated for explanatory, hence non-limitative purposes in the attached drawings.

### BRIEF DESCRIPTION OF FIGURES

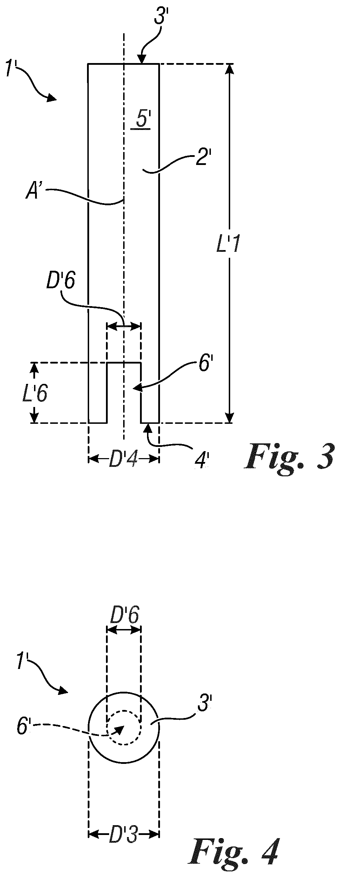

Figures 3 and 4 are not according to the invention and are present for illustration purposes only.
Figure 1 is a side view of a first embodiment of the device for closing a trocar site;
figure 2 is a view along the longitudinal axis of the device of figure 1;
figure 3 is a side view of a second embodiment of the device according to the invention; and
figure 4 is a view along the longitudinal axis of the device of figure 3.

### DETAILED DESCRIPTION OF THE INVENTION

With reference to the mentioned figures, a device 1 for closing a trocar site is shown, the device 1 comprising a body 2 made from a resorbable and sterile, biocompatible polymer.

The present invention refers to a device 1 consisting of a body 2 made from a resorbable, sterile and biocompatible polymer. The body 2 features an elongate shape along a longitudinal axis A and is provided with a first extremal surface 3, with a second extremal surface 4, and with at least one longitudinal surface 5 which extends along the longitudinal axis A between the first extremal surface 3 and the second extremal surface 4. At least a portion of the body 2, taken longitudinally between the first extremal surface 3 and the second extremal surface 4, is solid. The body 2 has a truncated-cone shape, and comprises a blind hole 6 in correspondence with one of the extremal surfaces 3, 4. According to the invention, the second extremal surface 4 has a diameter D4 smaller than the diameter D3 of the first extremal surface 3, and the blind hole 6 is cut in correspondence of said second extremal surface 4.

For instance, the biocompatible polymer (or biopolymer) can be selected from biocompatible polymers of animal origin (for instance gelatin, collagen, chitosan), biocompatible polymers of vegetal origin (for instance alginate, cellulose, polylactic acid), and synthetic biocompatible polymers (for instance polycaprolactone). Preferably, the biocompatible polymer is of animal origin.

According to an equally preferred aspect, the body 2 is made of a hemostatic sponge.

In the preferred embodiment of the device 1, the body 2 is made of a hemostatic gelatin sponge.

The hemostatic gelatin sponge is a bio-polymeric material particularly suitable for surgical use, in that it is biocompatible and it is quickly re-absorbable, on average within few weeks. In addition, such material is capable of promoting a platelet aggregation to implement the hemostasis. The hemostatic sponge has preferably a porosity greater than 60%, preferably ranging from 70% to 99%. It is capable of absorbing more than thirty-five times its own weight. Preferably, the density of the hemostatic sponge is of from 0,015 to 0,035 g/cm³; more preferably, the density is of from 0.020 to 0.030 g/cm³.

By solid portion of a body we mean, in this context, a portion of a body that is not crossed by a through hole.

The provision of at least a portion of the body 2 that is solid allows to obtain a device 1 that is more absorbing, that expands better after being introduced into the trocar site, and consequently that performs better.

It is preferred that at least half of the body 2, taken longitudinally, be solid. The body 2 might even be totally solid, and consequently the two extremal surfaces 3, 4 are solid surfaces.

By solid surface, in this context, we mean a surface deprived of holes.

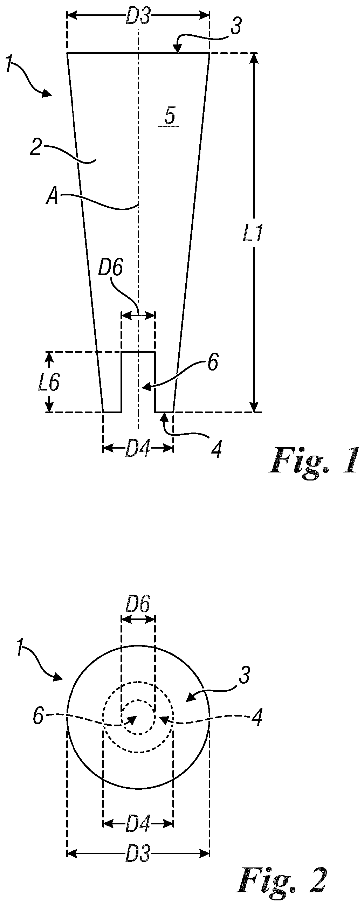

The example in figure 1 shows that it is advantageous that at least one of the extremal surfaces 3, 4 be solid.

Advantageously, the body 2 features the shape of a solid of rotation about the longitudinal axis A. In other words, it is preferred that the body 2 features a cross section, taken transversally to the longitudinal axis A, that is circularly shaped. More specifically, the cross section features a circular shape if taken perpendicularly to the longitudinal axis A. In this way, the device 1 is easier to introduce into trocars, which usually also feature a circular cross section. In addition, it features a greater contact surface with the organs internally to the abdominal cavity.

However, the body 2 might also feature a different cross section, for instance octagonal or the like, without prejudice to the achievement of the aimed result.

The maximum transversal dimension, i.e., the diameter in the example here illustrated, of the cross section of the body 2 might vary, either regularly or irregularly, along the axis A between the extremal surfaces 3, 4. Likewise, the first and second extremal surfaces 3, 4 might feature respective transversal dimensions D3, D4 different from each other.

The transversal dimension D3 of the first extremal surface 3 is different from the transversal dimension D4 of the second extremal surface 4. The transversal dimension D3 is greater than the transversal dimension D4 (D3 > D4).

Preferably the transversal dimension of the cross section of the body 2 varies regularly between the first extremal surface 3 and the second extremal surface 4. For example, the body 2 features a truncated-cone shape, as in the preferred embodiment illustrated in figures 1 and 2.

Typically, the body 2 features a length L1, taken between the first and second extremal surfaces 3, 4, greater than 40 mm, preferably ranging from 45 to 75 mm, more preferably from 55 to 65 mm, and even more preferably it is substantially equal to 60 mm. Devices might also be provided with lengths in the order of 150 mm, for instance, from which two separate devices 1 can be obtained.

The transversal dimensions of the body 2 are variable from one model to another depending on the dimension of the trocar used, and consequently also on the dimension of the incision to be closed. For example, considering a preferred embodiment (i.e. the one featuring a circular cross section) the diameter D3 ranges from 10 to 40 mm and the diameter D4 of the second extremal surface 4 ranges from 5 to 15 mm.

As an example, it is possible to think to a body 2 wherein the diameter D3 of the first extremal surface 3 equals 30 mm and the diameter D4 of the second extremal surface 4 equals 12 mm, or the diameter D3 of the first extremal surface 3 equals 24 mm and the diameter D4 of the second extremal surface 4 equals 10 mm, or the diameter D3 of the first extremal surface 3 equals 20 mm and the diameter D4 of the second extremal surface 4 equals 8 mm.

Should the body 2 feature a non-circular cross section, the value of the maximum transversal dimension is to be meant similar to those indicated here above.

It is advantageous to have the diameter D3 of the first extremal surface 3 substantially equal to twice the diameter of the trocar used in the surgical operation. Typically, considering a trocar having a diameter of 5 mm, a device 1 is provided whose first extremal surface 3 has a diameter D3 of 10 mm, for a trocar having a diameter of 10 mm the diameter D3 of the first extremal surface 3 will be 20 mm, and for a trocar having a diameter of 15 mm the device 1 should preferably have a diameter D3 of the first extremal surface 3 equal to 30 mm. The latter version represents the preferred size of the device 1 under consideration.

It is also advantageous for the device 1 to comprise, in correspondence with either extremal surfaces 3 or 4, a blind hole 6. The function of such blind hole 6 is to help in inserting the device 1 through the trocar, as it will be better explained below.

Preferably, the blind hole 6 is parallel to the longitudinal axis A of the device 1. Even more preferably, the blind hole 6 is coaxial to the same longitudinal axis A of the device 1.

It is particularly advantageous that such blind hole 6 features a cylindrical shape. The transversal section of the blind hole might be polygonal, but a circular cross section is preferred. The maximum lateral dimension D6 of the blind hole 6 (i.e. its inner diameter, should it be cylindrical) ranges from 2 to 13 mm, preferably from 2.5 to 10 mm, and even more preferably from 3 to 5 mm. In the preferred embodiment of the device 1, the maximum lateral dimension D6 of the blind hole 6 equals 4 mm.

Obviously, the hole 6, being blind, has a length L6 shorter than the length L1 of the body 2. More preferably the length L6 of the blind hole 6 is shorter than half the length L1 of the body 2 of the device 1. Even more preferably the length L6 of the blind hole 6 is shorter than one fourth of the length L1 of the body 2 of the device 1. In the example here illustrated, the length L6 of the blind hole 6 substantially equals one sixth of the length L1 of the device 1 (L6=L1/6).

In the example illustrated in figures 1 and 2, the blind hole has a length L6 substantially equal to 10 mm, and its inner diameter D6 substantially equals 4 mm. Contrary to what has been said for the lateral dimensions of the body 2, the dimensions of the blind hole 6 are not selected as a function of the lateral dimension of the trocar, hence of the trocar site to be closed.

It is worth noting that the blind hole 6 is properly provided in the second extremal surface 4, i.e. the one featuring a diameter D4 smaller than the diameter D3 of the first extremal surface 3.

The device 1 of the invention is typically employed as it follows.

A device 1 is supplied to a surgeon closed in a sterile and hermetically sealed packaging.

Upon its use, the surgeon opens the packaging and takes the device 1 out, then compresses it in order to introduce it into the trocar. When using a truncated-cone shaped device, as illustrated in figures 1 and 2, the extremal surface 3 featuring the greater diameter D3 shall be inserted first.

Once the device 1 is inserted into the trocar, it is pushed in through the trocar, with the help of an appropriate tool, typically a sterilized rod. Then the surgeon adjusts the positioning of the device 1 internally to the incision, by looking at it either directly or through the laparoscopic device used for the surgical operation.

To get a greater accuracy in guiding the device 1 through the trocar and internally to the incision, it is convenient that the surgeon inserts the end of his/her tool into the blind hole 6 cut in the second extremal surface 4.

Once the device 1 is fully inserted into the incision, the surgeon can remove the trocar. At this point, the outer part of the device 1, i.e. the second extremal surface 4, is secured at a depth of approximately 1 cm into the subcutaneous tissue, so as to prevent the device 1 from migrating in the intra-abdominal cavity.

Having inserted the device 1 into the trocar site, the latter is closed.

Devices according to the invention have been tested by experienced surgeons in clinical practice.

Devices according to the invention, having a truncated-conical shape and a blind hole cut at one of the extremal surfaces, provide better maneuverability compared to the devices of the prior art, leading to a significant reduction of the intervention time, improvement of the repeatability of the procedure and a better closure of the surgical site.

With the devices of the invention, having a truncated-conical shape and a blind hole cut at one of the extremal surfaces, the intra-abdominal migration of the plug is prevented.

Furthermore, devices made of high density polymers provide the best maneuverability.

As with the first embodiment, the transversal dimension of the body 2' is variable depending on the size of the trocar used, hence of the dimension of the incision made to be closed too. For example, the diameter D'3, D'4 typically ranges from 5 to 15 mm. The dimensions of the blind hole 6' (its length L'6 and its diameter D'6) don't vary with respect to the first embodiment.

Devices according to the invention allow to close the incisions, the so-called trocar sites, in a simpler manner and more safely for patients, especially when they are located on the abdominal wall. The present solution is particularly effective, in that the device creates a fibrosis which closes the incision.

Thanks to the body of the device being particularly porous, bleeding is blocked safely and quickly. In addition, the combination of the spongy material with the dimensions used is such that the device, once arranged inside the incision, expands and presses the inner walls of the trocar site, thus fostering the hemostasis. Such expansion and its consequent pressure are obtained thanks to the at least a portion of the body (taken longitudinally to the axis A, A') being solid.

It is also worth underlining that the polymeric material used makes it possible for the device to be soaked with pharmaceutical substances at choice, which allows to improve the hemostaticity and cicatrization properties of the device itself (for instance thrombin and fibrin), or to improve the antiseptic treatment in the post-operative phase (for instance with local antibiotics).

Thanks to the device according to the invention, it is now possible to close trocar sites located in areas of the body, in particular of the abdominal area, that are difficult to access and consequently in which suture is complicate to perform.

It is also worth outlining that devices made from a sponge of a biocompatible polymer already exist for closing trocar sites, but they have different shapes and are used for closing trocar sites in other areas of the human body. It is the first time a biocompatible polymer sponge, and in particular a resorbable and sterile, hemostatic gelatin sponge, is used in intra-abdominal cavity surgery, and also in bariatric surgery.

### EXAMPLE

The compressive strength of devices made of hemostatic sponge of various density, according to preferred embodiments of the invention, has been tested. A set-up has been used, where a constant force is applied perpendicular to one extremal surface of the device, directed along the longitudinal axis (A) of the device towards the opposite extremal surface. The force is measured with a dynamometer, until the maximum compression of the device is reached.

Through the measurement of the initial and final length of the longitudinal axis of the devices, under compression, the deformation of the various samples of different density has been calculated. Devices of low, medium, or high density (LD, MD and HD, respectively) have been each tested twice. The density (*ρ*) of each sample is reported in table 1, together with the results of the test.

**Table 1**

| Sample | ρ(g/cm³) | ε (-) | σ (N/cm2) | E (MPa) | Mean E (MPa) |
|---|---|---|---|---|---|
| LD | 0,019 | 0,350 | 5,00 | 0, 14 | 0, 146 |
| LD | 0,019 | 0,333 | 5,00 | 0,15 | |
| MD | 0,025 | 0,375 | 5, 47 | 0, 15 | 0,135 |
| MD | 0,025 | 0,500 | 6,25 | 0, 12 | |
| HD | 0,030 | 0,367 | 10,00 | 0,27 | 0,262 |
| HD | 0,030 | 0,435 | 10, 93 | 0,25 | |

The deformation value (*ε*), the stress value (*σ*) applied to reach the maximum sample deformation, the Elastic modulus (*E*) and the mean elastic modulus (Average E) are reported.

Devices having a density of about 0.030 g/cm³ are more rigid and provide the best maneuverability.

## Claims

1. A device for closing a trocar site, said device (1) consisting of a body (2) made from a resorbable, sterile and biocompatible polymer, wherein said body (2) features an elongate shape along a longitudinal axis (A) and is provided with a first extremal surface (3), with a second extremal surface (4), and with at least one longitudinal surface (5) which extends along said longitudinal axis (A) between said first extremal surface (3) and said second extremal surface (4), wherein at least a portion of said body (2), taken longitudinally between said first extremal surface (3) and said second extremal surface (4), is solid, wherein said body (2) has a truncated-cone shape, and said body (2) comprises a blind hole (6) in correspondence with one of the extremal surfaces (3, 4), the device (1) being **characterized in that** the second extremal surface (4) has a diameter (D4) smaller than the diameter (D3) of the first extremal surface (3), and wherein the blind hole (6) is cut in correspondence of said second extremal surface (4).

2. The device according to claim 1, wherein the body (2) is made from hemostatic sponge.

3. The device according to claim 1 or 2, wherein said polymer is of animal origin.

4. The device according to one or more of the previous claims, wherein the body (2) has the shape of a solid of rotation about the longitudinal axis (A).

5. The device according to one or more of the previous claims, wherein said blind hole (6) is parallel to the longitudinal axis (A) of said device (1).

6. The device according to one or more of the previous claims wherein that the blind hole (6) is coaxial to the longitudinal axis (A) of said device (1).

7. The device according to one or more of claims 2 to 6, **characterized in that** the hemostatic sponge has a porosity greater than 60%.

8. The device according to one or more of the previous claims, wherein said blind hole (6) has a circular or polygonal cross-section.

9. The device of one or more of the previous claims, wherein the length (L6) of the blind hole (6) is shorter than half the length (LI) of the body (2) of the device (1).

## Patentansprüche

1. Vorrichtung zum Verschließen einer Trokarstelle, wobei die Vorrichtung (1) aus einem Körper (2) aus einem resorbierbaren, sterilen und biokompatiblen Polymer besteht, wobei der Körper (2) eine längliche Form entlang einer Längsachse (A) aufweist und mit einer ersten Endfläche (3) sowie einer zweiten Endfläche (4) versehen ist, sowie mit mindestens einer Längsfläche (5), die sich entlang der Längsachse (A) zwischen der ersten Endfläche (3) und der zweiten Endfläche (4) erstreckt, wobei mindestens ein Abschnitt des Körpers (2), der in Längsrichtung zwischen der ersten Endfläche (3) und der zweiten Endfläche (4) liegt, massiv ist, wobei der Körper (2) eine Kegelstumpfform aufweist, und der Körper (2) ein Sackloch (6) aufweist, das mit einer der Endflächen (3, 4) korrespondiert, wobei die Vorrichtung (1) **dadurch gekennzeichnet ist, dass** die zweite Endfläche (4) einen Durchmesser (D4) aufweist, der kleiner ist als der Durchmesser (D3) der ersten Endfläche (3), und wobei das Sackloch (6) in Übereinstimmung mit der zweiten Endfläche (4) ausgebildet ist.

2. Vorrichtung nach Anspruch 1, wobei der Körper (2) aus einem hämostatischen Schwamm besteht.

3. Vorrichtung nach Anspruch 1 oder 2, wobei das Polymer tierischen Ursprungs ist.

4. Vorrichtung nach einem oder mehreren der vorstehenden Ansprüche, wobei der Körper (2) die Form eines Rotationskörpers um die Längsachse (A) aufweist.

5. Vorrichtung nach einem oder mehreren der vorstehenden Ansprüche, wobei das Sackloch (6) parallel zur Längsachse (A) der Vorrichtung (1) verläuft.

6. Vorrichtung nach einem oder mehreren der vorstehenden Ansprüche, wobei das Sackloch (6) koaxial zur Längsachse (A) der Vorrichtung (1) ist.

7. Vorrichtung nach einem oder mehreren der Ansprüche 2 bis 6, **dadurch gekennzeichnet, dass** der hämostatische Schwamm eine Porosität von mehr als 60 % aufweist.

8. Vorrichtung nach einem oder mehreren der vorstehenden Ansprüche, wobei das Sackloch (6) einen kreisförmigen oder polygonalen Querschnitt aufweist.

9. Vorrichtung nach einem oder mehreren der vorstehenden Ansprüche, wobei die Länge (L6) des Sacklochs (6) kürzer ist als die Hälfte der Länge (L1) des Körpers (2) der Vorrichtung (1).

## Revendications

1. Dispositif destiné à fermer un site de trocart, ledit dispositif (1) étant constitué d'un corps (2) fabriqué à partir d'un polymère résorbable, stérile et biocompatible, dans lequel ledit corps (2) présente une forme allongée le long d'un axe longitudinal (A) et est muni d'une première surface d'extrémité (3), d'une deuxième surface d'extrémité (4), et d'au moins une surface longitudinale (5) qui s'étend le long dudit axe longitudinal (A) entre ladite première surface d'extrémité (3) et ladite deuxième surface d'extrémité (4), dans lequel au moins une partie dudit corps (2), prise longitudinalement entre ladite première surface d'extrémité (3) et ladite deuxième surface d'extrémité (4), est pleine, dans lequel ledit corps (2) a une forme de cône tronqué, et ledit corps (2) comprend un trou borgne (6) en correspondance avec l'une des surfaces d'extrémité (3, 4), le dispositif (1) étant **caractérisé en ce que** la deuxième surface d'extrémité (4) a un diamètre (D4) inférieur au diamètre (D3) de la première surface d'extrémité (3), et dans lequel le trou borgne (6) est découpé en correspondance avec ladite deuxième surface d'extrémité (4).

2. Dispositif selon la revendication 1, dans lequel le corps (2) est constitué d'une éponge hémostatique.

3. Dispositif selon la revendication 1 ou 2, dans lequel ledit polymère est d'origine animale.

4. Dispositif selon l'une ou plusieurs des revendications précédentes, dans lequel le corps (2) a la forme d'un solide de révolution autour de l'axe longitudinal (A).

5. Dispositif selon une ou plusieurs des revendications précédentes, dans lequel ledit trou borgne (6) est parallèle à l'axe longitudinal (A) dudit dispositif (1).

6. Dispositif selon l'une ou plusieurs des revendications précédentes, dans lequel le trou borgne (6) est coaxial à l'axe longitudinal (A) dudit dispositif (1).

7. Dispositif selon l'une ou plusieurs des revendications 2 à 6, **caractérisé en ce que** l'éponge hémostatique présente une porosité supérieure à 60 %.

8. Dispositif selon l'une ou plusieurs des revendications précédentes, dans lequel ledit trou borgne (6) présente une section transversale circulaire ou polygonale.

9. Dispositif selon l'une ou plusieurs des revendications précédentes, dans lequel la longueur (L6) du trou borgne (6) est inférieure à la moitié de la longueur (L1) du corps (2) du dispositif (1).
